(19) **Europäisches Patentamt European Patent Office Office européen des brevets**

(11) **EP 3 452 133 B1**

(12) **EUROPEAN PATENT SPECIFICATION**

(45) Date of publication and mention of the grant of the patent:
**08.04.2020 Bulletin 2020/15**

(51) Int Cl.:
**A61M 1/02** *(2006.01)* **A61M 1/36** *(2006.01)*
**B01D 21/28** *(2006.01)*

(21) Application number: **17721668.6**

(22) Date of filing: **04.05.2017**

(86) International application number:
**PCT/EP2017/060718**

(87) International publication number:
**WO 2017/191289 (09.11.2017 Gazette 2017/45)**

(54) **MULTIPLE BLOOD BAG SYSTEM**

MEHRTEILIGES BLUTBEUTELSYSTEM

SYSTÈME À POCHES DE SANG MULTIPLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2016 PCT/EP2016/060090**

(43) Date of publication of application:
**13.03.2019 Bulletin 2019/11**

(73) Proprietor: **Aenitis Technologies**
**77290 Mitry-Mory (FR)**

(72) Inventors:
• **VINCENT, Emmanuel**
**77290 Mitry-Mory (FR)**
• **BOHEC, Pierre**
**28000 Chartres (FR)**
• **GACHELIN, Jérémie**
**94110 Arcueil (FR)**

(74) Representative: **Icosa**
**83 avenue Denfert-Rochereau**
**75014 Paris (FR)**

(56) References cited:
**US-A1- 2008 181 828     US-A1- 2010 078 384**
**US-A1- 2015 110 763**

**Description**

**FIELD OF INVENTION**

The present invention relates to the field of blood collection and fractionation. Especially the present invention relates to a multiple blood bag system comprising an acoustic sorter for fractionating whole blood into its components. The present invention also relates to a method for preparing all kinds of blood products using a multiple blood bag system.

**BACKGROUND OF INVENTION**

Such blood bag system for fractionating blood usually consists of a first collecting bag used for collecting whole blood and one or more sub-bags used for fractionating blood into separated blood products. Plastic tubing couples said bags together to form a so-called closed multiple blood bag system. Typical system comprises four or five blood bags in fluid communication such that once whole blood is introduced into the system, the whole blood or its components may be moved from one bag to another by external manipulation of valves, and the like, thereby avoiding contamination. Depending on the configuration, the blood bag system may also include a white blood cells removing filter, downstream the whole blood bag.

Document US 2008/181828 discloses a multiple blood bag system for fractioning blood.

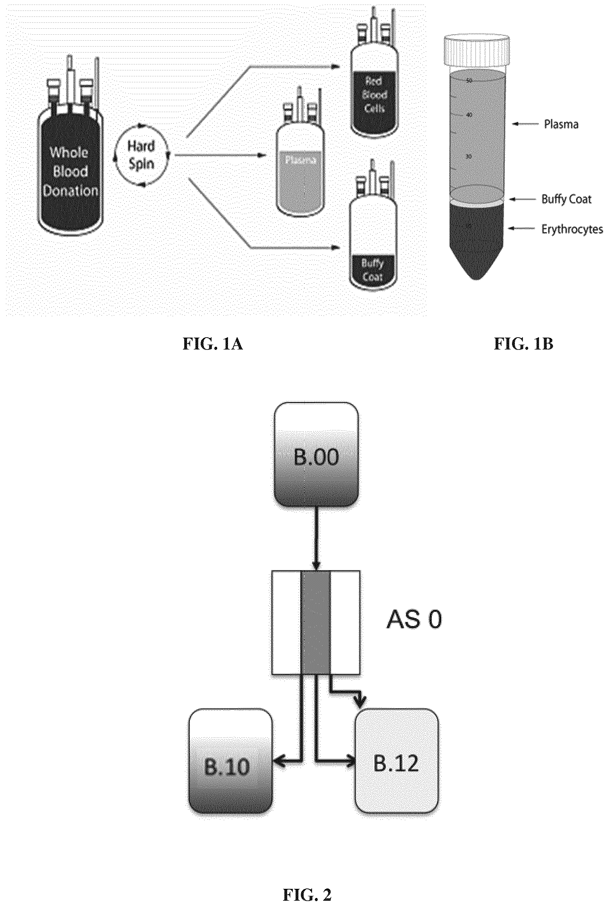

In a typical multiple blood bag system application, whole blood is collected into a first bag and all connected blood bags, which are usually empty, are placed in a centrifuge. The bag contents are then centrifuged to separate whole bloods into its components (see figure 1A). By manipulating a valve, which is typically a frangible valve within the system, the blood products of the whole blood may then be transferred into one of the others blood bags, possibly for further processing. Said blood products may be red blood cell concentrate (hereinafter referred to as RBC), platelet concentrate (hereinafter referred to as PC), blood plasma (hereinafter referred to as BP) including category 1 and category 2 blood plasma, or the like.

Centrifugation of whole blood in typical blood processing raises several issues. The high rotation speed allows stratification of the whole blood (see figure 1B) but also induce strong shear on the cells thereby activating PC. Due to centrifugation of the whole blood, between 20 to 40% of the platelets collected may indeed be activated. It strongly affects the efficiency of the PC when injected to a patient and also reduce its storage time to no more than 5 days. Other blood products, such as RBC, may also be affected by the high rotation speed. Furthermore, fractionation of whole blood by centrifugation lasts up to 7 hours and requires several manuals steps and at least two centrifugations.

The present invention aims at overcoming the drawbacks of the prior art by providing a method for preparing all kinds of blood products and an easy-to-use, closed, disposable, sterile, multiple blood bag system enabling production of all the blood products within a single system, limiting manual operations and also avoiding any centrifugation step.

**DEFINITIONS**

In the present invention, the following terms have the following meanings:

- **"About"** preceding a figure means plus or minus 10% of the value of said figure.

- **"Blood products"** refer to specific components obtained from whole blood collected from a donor; said specific components may be red blood cell concentrate, white blood cell concentrate, blood plasma or platelet concentrate.

- **"Closed system"** refers to a system that is isolated from its surroundings by boundaries that admits no transfer of matter across it.

- **"Disposable system"** refers to a system configured to be thrown away after a single use.

- **"Sterile system"** refers to an aseptic system free from living germs or microorganisms.

- **"Platelets activation"** refers to a series of cascading responses which allow blood platelets to react to an injury by shape change, adhesiveness, aggregation, and release reaction. Blood plasma increases platelets activation.

**DETAILED DESCRIPTION**

The following detailed description will be better understood when read in conjunction with the drawings.

The present invention relates to a method for high throughput preparation of blood products.

The present invention especially relates to a method for high throughput preparation of blood products to be used for blood transfusion comprising the following steps:

- providing a closed disposable sterile multiple blood bag system comprising:

  - a fluid collecting bag **B.00** comprising at least one outlet port, said fluid collecting bag containing whole blood obtained from an individual;
  - first and second sampling bags **B.10, B.12,** each comprising at least one inlet port and at least one outlet port;
  - first means for transferring fluid **AS0** from the first collecting bag **B.00** to the sampling bags **B.10, B.12,** wherein the first means for transferring fluid **AS0** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength λ within the channel;

wherein the thickness of the channel is equal to λ/2; the at least one outlet port of the fluid collecting bag **B.00** is sterilely and fluidly connected to the at least one inlet of the first means for transferring fluid **AS0** and the at least first and second outlets of the first means for transferring fluid **AS0** are sterilely and fluidly connected to the at least one inlet port of respectively the first and second sampling bags **B.12, B.10;**

- applying acoustic field inside the channel of the means for transferring fluid **AS0** by means of the acoustic wave generator;
- transferring the content of the fluid collecting bag **B.00** in the first means for transferring fluid **AS0;** and
- collecting blood cells in the first sampling bag **B.10** and blood plasma in the second sampling bag **B.12.**

According to one embodiment, the closed disposable sterile multiple blood bag system further comprises a first buffer bag comprising at least one outlet port, said first buffer bag containing a buffer medium, and said at least one outlet port is sterilely and fluidly connected to the at least one inlet of the first means for transferring fluid **AS0.** According to said embodiment, the method comprises the step of transferring the content of the fluid collecting bag **B.00** and the first buffer bag in the first means for transferring fluid **AS0.**

According to a further embodiment, the method further comprises fractionation of blood cells into platelet concentrate and red blood cell concentrate. According to said embodiment, the closed disposable sterile multiple blood bag system further comprises:

- a second buffer bag **B.11** comprising at least one outlet port; said second buffer bag **B.11** containing a buffer medium;

- third and fourth sampling bags **B.20, B.22,** each comprising at least one inlet port and at least one outlet port;
- second means for transferring fluid **AS1** from the first sampling bag **B.10** to the third and fourth sampling bags **B.20, B.22,** wherein the second means for transferring fluid **AS1** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength λ within the channel;

wherein the thickness of the channel is equal to λ/2; the at least one outlet port of the first sampling bag **B.10** is fluidly and sterilely connected to the at least one inlet of the second means for transferring fluid **AS1,** the at least first and second outlets of the second means for transferring fluid **AS1** are fluidly and sterilely connected to the inlet port of respectively the third and fourth sampling bags **B.20, B.22,** and the at least one outlet port of the second buffer bag **B.11** is fluidly and sterilely connected to the at least one inlet of the second means for transferring fluid **AS1.**

According to said embodiment, the method further comprises the following steps:

- applying acoustic field inside the channel of the second means for transferring fluid **AS1** by means of the acoustic wave generator;
- transferring the content of the first sampling bag **B.10** and the second buffer bag **B.11** in the second means for transferring fluid **AS1;** and
- collecting red blood cell concentrate in the third sampling bag **B.20** and platelet concentrate in the fourth collecting bag **B.22.**

According to one embodiment, the white blood cells are removed using a white blood cells removing filter located between the first outlet port of the second means for transferring fluid **AS1** and the at least one inlet port of the third sampling bag **B.20.**

The present invention also relates to a method for high throughput preparation of blood products to be used for blood transfusion comprising the following steps:

- providing a closed disposable sterile multiple blood bag system comprising:

  - a fluid collecting bag **B.00** comprising at least one outlet port, said fluid collecting bag containing whole blood obtained from an individual;
  - first and second sampling bags **B.10, B.12,** each comprising at least one inlet port and at least one outlet port;
  - first means for transferring fluid **AS0** from the first collecting bag **B.00** to the sampling bags **B.10, B.12,** wherein the first means for transferring fluid **AS0** comprises:

    • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
    • at least one inlet in fluid communication with the channel;
    • at least first and second outlets in fluid communication with the channel, the first outlet being separated, on the second transverse axis (z), from the second outlet; and
    • optionally at least one acoustic wave generator for generating acoustic wave having a wavelength λ within the channel;

    wherein the at least one outlet port of the fluid collecting bag **B.00** is sterilely and fluidly connected to the at least one inlet of the first means for transferring fluid **AS0** and the at least first and second outlets of the first means for transferring fluid **AS0** are sterilely and fluidly connected to the at least one inlet port of respectively the first and second sampling bags **B.12, B.10;**

- applying acoustic field inside the channel of the means for transferring fluid **AS0** by means of the acoustic wave generator;
- transferring the content of the fluid collecting bag **B.00** in the first means for transferring fluid **AS0;** and
- collecting blood cells in the first sampling bag **B.10** and blood plasma in the second sampling bag **B.12.**

The present invention also relates to a method for high throughput preparation of blood products to be used for blood transfusion, the method comprising the following steps:

- providing a closed disposable sterile multiple blood bag system comprising:
- a fluid collecting bag **B.00** comprising at least one outlet port, said fluid collecting bag containing whole blood obtained from an individual;

- first and second sampling bags **(B.10, B.12),** each comprising at least one inlet port and at least one outlet port;
- a second buffer bag **B.11** comprising at least one outlet port; said second buffer bag **B.11** containing a buffer medium;
- third and fourth sampling bags **(B.20, B.22),** each comprising at least one inlet port and at least one outlet port;
- first means for transferring fluid **AS0** from the first collecting bag **B.00** to the sampling bags **(B.10, B.12),** and second means for transferring fluid **AS1** from the first sampling bag **B.10** to the third and fourth sampling bags **(B.20, B.22),** wherein the first and second means for transferring fluid **(AS0, AS1)** each comprise:

  • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
  • at least one inlet in fluid communication with the channel;
  • at least first and second outlets in fluid communication with the channel, the first outlet being separated, on the second transverse axis (z), from the second outlet; and

the at least one outlet port of the fluid collecting bag **B.00** is sterilely and fluidly connected to the at least one inlet of the first means for transferring fluid **AS0** and the at least first and second outlets of the first means for transferring fluid **AS0** are sterilely and fluidly connected to the at least one inlet port of respectively the first and second sampling bags **(B.12, B.10);** the at least one outlet port of the first sampling bag **B.10** is fluidly and sterilely connected to the at least one inlet of the second means for transferring fluid **AS1,** the at least first and second outlets of the second means for transferring fluid **AS1** are fluidly and sterilely connected to the inlet port of respectively the third and fourth sampling bags **(B.20, B.22),** and the at least one outlet port of the second buffer bag **B.11** is fluidly and sterilely connected to the at least one inlet of the second means for transferring fluid **AS1;** and

- applying acoustic field inside the channel of the means for transferring fluid **AS0** by means of the acoustic wave generator;
- transferring the content of the fluid collecting bag **B.00** in the first means for transferring fluid **AS0;** and
- collecting blood cells in the first sampling bag **B.10**

and blood plasma in the second sampling bag **B.12;**

- applying acoustic field inside the channel of the second means for transferring fluid **AS1** by means of the acoustic wave generator;
- transferring the content of the first sampling bag **B.10** and the second buffer bag **B.11** in the second means for transferring fluid **AS1;** and
- collecting red blood cell concentrate in the third sampling bag **B.20** and platelet concentrate in the fourth collecting bag **B.22.**

According one embodiment, means for transferring fluid **AS0** and **AS1** each comprise at least one acoustic wave generator for generating acoustic wave having a wavelength λ within the channel. In said embodiment, the thickness of the channel of each means for transferring fluid is equal to $\frac{\lambda}{2}$, or equal to a multiple of $\frac{\lambda}{2}$. According to an alternative embodiment, an acoustic wave generator is provided in vicinity of the channels of means for transferring fluid **AS0** and **AS1** for generating acoustic wave having a wavelength λ within the channel. In said embodiment, the acoustic wave generator is not a part of the closed disposable sterile multiple blood bag system.

According to one embodiment, the closed disposable sterile multiple blood bag system comprises at least two first means for transferring fluid **AS0** from the first collecting bag **B.00** to the sampling bags **(B.10, B.12),** and at last two second means for transferring fluid **AS1** from the first sampling bag **B.10** to the third and fourth sampling bags **(B.20, B.22).**

According to one embodiment, the method is a method for high throughput preparation of blood products to be used for blood transfusion, the method comprising the following steps:

- providing a closed disposable sterile multiple blood bag system comprising:

  - a fluid collecting bag **B.00** comprising at least one outlet port, said fluid collecting bag containing whole blood obtained from an individual;
  - first and second sampling bags **(B.10, B.12),** each comprising at least one inlet port and at least one outlet port;
  - a second buffer bag **B.11** comprising at least one outlet port; said second buffer bag **B.11** containing a buffer medium;
  - third and fourth sampling bags **(B.20, B.22),** each comprising at least one inlet port and at least one outlet port;
  - first means for transferring fluid **AS0** from the first collecting bag **B.00** to the sampling bags **(B.10, B.12),** and second means for transferring fluid **AS1** from the first sampling bag **B.10** to the third and fourth sampling bags **(B.20, B.22),** wherein the first and second means for transferring fluid **(AS0, AS1)** each comprise:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength λ within the channel;

wherein the thickness of the channel is equal to λ/2 or is equal to a multiple of λ/2; the at least one outlet port of the fluid collecting bag **B.00** is sterilely and fluidly connected to the at least one inlet of the first means for transferring fluid **AS0** and the at least first and second outlets of the first means for transferring fluid **AS0** are sterilely and fluidly connected to the at least one inlet port of respectively the first and second sampling bags **(B.12, B.10);** the at least one outlet port of the first sampling bag **B.10** is fluidly and sterilely connected to the at least one inlet of the second means for transferring fluid **AS1,** the at least first and second outlets of the second means for transferring fluid **AS1** are fluidly and sterilely connected to the inlet port of respectively the third and fourth sampling bags **(B.20, B.22),** and the at least one outlet port of the second buffer bag **B.11** is fluidly and sterilely connected to the at least one inlet of the second means for transferring fluid **AS1;** and

- applying acoustic field inside the channel of the means for transferring fluid **AS0** by means of the acoustic wave generator;
- transferring the content of the fluid collecting bag **B.00** in the first means for transferring fluid **AS0;** and
- collecting blood cells in the first sampling bag **B.10** and blood plasma in the second sampling bag **B.12;**
- applying acoustic field inside the channel of the second means for transferring fluid **AS1** by means of the acoustic wave generator;
- transferring the content of the first sampling bag **B.10** and the second buffer bag **B.11** in the second means for transferring fluid **AS1;** and

- collecting red blood cell concentrate in the third sampling bag **B.20** and platelet concentrate in the fourth collecting bag **B.22.**

According to one embodiment wherein white blood cells removing filter is not implemented, the third sampling bag **B.20** comprises red blood cell concentrate and white blood cell concentrate. According to said embodiment, the content of the third sampling bag may be fractionated into red blood cell concentrate and white blood cell concentrate. According to said embodiment, the closed disposable sterile multiple blood bag system further comprises:

- a third buffer bag **B.21** comprising at least one outlet port; said third buffer bag **B.21** containing a buffer medium;
- fifth and sixth sampling bags **B.30, B.31,** each comprising at least one inlet port and at least one outlet port;
- third means for transferring fluid **AS2** from the third sampling bag **B.20** to the fifth and sixth sampling bags **B.30, B.31,** wherein the third means for transferring fluid **AS2** comprise:

  • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
  • at least one inlet in fluid communication with the channel;
  • at least first and second outlets in fluid communication with the channel, the first outlet being separated, on the second transverse axis (z), from the second outlet; and
  • at least one acoustic wave generator for generating acoustic wave having a wavelength λ within the channel;

wherein the thickness of the channel is equal to λ/2; the at least one outlet port of the third sampling bag **B.20** is fluidly and sterilely connected to the at least one inlet of the third means for transferring fluid **AS2,** the at least first and second outlets of the third means for transferring fluid **AS2** are fluidly and sterilely connected to the inlet port of respectively the fifth and sixth sampling bags **B.30, B.31,** and the outlet of the third buffer bag is fluidly and sterilely connected to the at least one inlet of the third means for transferring fluid **AS2.**

According to said embodiment, the closed disposable sterile multiple blood bag system further comprises:

- a third buffer bag **B.21** comprising at least one outlet port; said third buffer bag **B.21** containing a buffer medium;
- fifth and sixth sampling bags **B.30, B.31,** each comprising at least one inlet port and at least one outlet port;
- third means for transferring fluid **AS2** from the third sampling bag **B.20** to the fifth and sixth sampling bags **B.30, B.31,** wherein the third means for transferring fluid **AS2** comprise:

  • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
  • at least one inlet in fluid communication with the channel;
  • at least first and second outlets in fluid communication with the channel, the first outlet being separated, on the second transverse axis (z), from the second outlet; and
  • at least one acoustic wave generator for generating acoustic wave having a wavelength λ within the channel;

wherein the at least one outlet port of the third sampling bag **B.20** is fluidly and sterilely connected to the at least one inlet of the third means for transferring fluid **AS2,** the at least first and second outlets of the third means for transferring fluid **AS2** are fluidly and sterilely connected to the inlet port of respectively the fifth and sixth sampling bags **B.30, B.31,** and the outlet of the third buffer bag is fluidly and sterilely connected to the at least one inlet of the third means for transferring fluid **AS2.**
According to said embodiment, the method further comprises the following steps:

- applying acoustic field inside the channel of the third means for transferring fluid **AS2** by means of the acoustic wave generator;
- transferring the content of the third sampling bag **B.20** and the third buffer bag **B.21** in the third means for transferring fluid **AS2;** and
- collecting red blood cell concentrate in the fifth sampling bag **B.30** and white blood cell concentrate in the sixth collecting bag **B.31.**

According to one embodiment, the platelet concentrate contains in the fourth sampling bag **B.22** may be further concentrated. According to said embodiment, the closed disposable sterile multiple blood bag system further comprises:

- a fourth buffer bag **B.23** comprising at least one outlet port; said fourth buffer bag **B.23** containing a buffer medium;

- seventh and eight sampling bags **B.32, B.33,** each comprising at least one inlet port and at least one outlet port;
- fourth means for transferring fluid **AS3** from the fourth sampling bag **B.22** to the seventh and eight sampling bags **B.32, B.33,** wherein the means for transferring fluid **AS3** comprises:

  • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
  • at least one inlet in fluid communication with the channel;
  • at least first and second outlets in fluid communication with the channel, the first outlet being separated, on the second transverse axis (z), from the second outlet; and
  • at least one acoustic wave generator for generating acoustic wave having a wavelength λ within the channel;

wherein the thickness of the channel is equal to $\frac{\lambda}{2}$;
the at least one outlet port of the fourth sampling bag **B.22** is fluidly and sterilely connected to the at least one inlet of the fourth means for transferring fluid **AS3,** the at least first and second outlets of the fourth means for transferring fluid **AS3** are fluidly and sterilely connected to the inlet port of respectively the seventh and eight sampling bags **B.32, B.33,** and the outlet of the fourth buffer bag **B.23** is fluidly and sterilely connected to the at least one inlet of the fourth means for transferring fluid **AS3.**

According to one embodiment, the closed disposable sterile multiple blood bag system further comprises:

- a fourth buffer bag **B.23** comprising at least one outlet port; said fourth buffer bag **B.23** containing a buffer medium;
- seventh and eight sampling bags **B.32, B.33,** each comprising at least one inlet port and at least one outlet port;
- fourth means for transferring fluid **AS3** from the fourth sampling bag **B.22** to the seventh and eight sampling bags **B.32, B.33,** wherein the means for transferring fluid AS3 comprises:

  • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
  • at least one inlet in fluid communication with the channel;
  • at least first and second outlets in fluid communication with the channel, the first outlet being separated, on the second transverse axis (z), from the second outlet; and
  • at least one acoustic wave generator for generating acoustic wave having a wavelength λ within the channel;

wherein the at least one outlet port of the fourth sampling bag **B.22** is fluidly and sterilely connected to the at least one inlet of the fourth means for transferring fluid **AS3,** the at least first and second outlets of the fourth means for transferring fluid **AS3** are fluidly and sterilely connected to the inlet port of respectively the seventh and eight sampling bags **B.32, B.33,** and the outlet of the fourth buffer bag **B.23** is fluidly and sterilely connected to the at least one inlet of the fourth means for transferring fluid **AS3.**

According to said embodiment, the method further comprises the following steps:

- applying acoustic field inside the channel of the fourth means for transferring fluid **AS3** by means of the acoustic wave generator;
- transferring the content of the fourth sampling bag **B.22** and the fourth buffer bag **B.23** in the fourth means for transferring fluid **AS3;** and
- collecting platelet concentrate in the seventh sampling bag **B.32.**

According to one embodiment, said steps of platelets concentration may be repeated as needed, preferably from 1 to 10 times.

According to one embodiment, the throughput of blood product preparation (i.e. the flow rate inside the means for transferring fluid **AS0, AS1, AS2, AS3** is ranging from 0.5mL/min to 100mL/min, from 0.5 to 20mL/min or about 20mL/min.

According to one embodiment, the method for preparation of blood product is not an apheresis.

According to one embodiment, the transfer of the content of the bag(s) in the means for transferring fluid is performed in any appropriate manner, such as gravity flow or active flow system (e.g. pumps) located on the external side or surface of the closed disposable sterile multiple blood bag system.

This invention also relates to a multiple bag system for fractionating a biological fluid into its components. The present invention especially relates to a closed disposable sterile multiple blood bag system for fractionating blood a depicted in figure 2.

Said closed disposable multiple sterile blood bag system for fractionating blood comprises:

- a fluid collecting bag **B.00** comprising at least one outlet port;
- first and second sampling bags **B.10, B.12,** each comprising at least one inlet port and at least one outlet port;
- first means for transferring fluid **AS0** from the fluid collecting bag **B.00** to the first and second sampling bags **B.10, B.12;**

wherein the first means for transferring fluid **AS0** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength $\lambda$ within the channel;

wherein the thickness of the channel is equal to $\lambda/2$; the at least one outlet port of the fluid collecting bag **B.00** is fluidly and sterilely connected to the at least one inlet of the first means for transferring fluid **AS0** and the at least first and second outlets of the first means for transferring fluid **AS0** are fluidly and sterilely connected to the at least one inlet port of respectively the first and second sampling bags **B.10, B.12.**

According to one embodiment, the closed disposable multiple sterile blood bag system for fractionating blood further comprises a first buffer bag comprising at least one outlet port; said first buffer bag containing a buffer medium, and the at least one outlet port of the first buffer bag is fluidly and sterilely connected to the at least one inlet of the first means for transferring fluid **AS0.**

According to one embodiment, the fluid collecting bag **B.00** comprises an inlet port which is fluidly and sterilely connected to a vein puncture needle.

According to one embodiment, the closed disposable sterile multiple blood bag system further comprises an initial sampling bag comprising an inlet port which is fluidly and sterilely connected to a vein puncture needle and an outlet port which is fluidly and sterilely connected to the fluid collecting bag **B.00.**

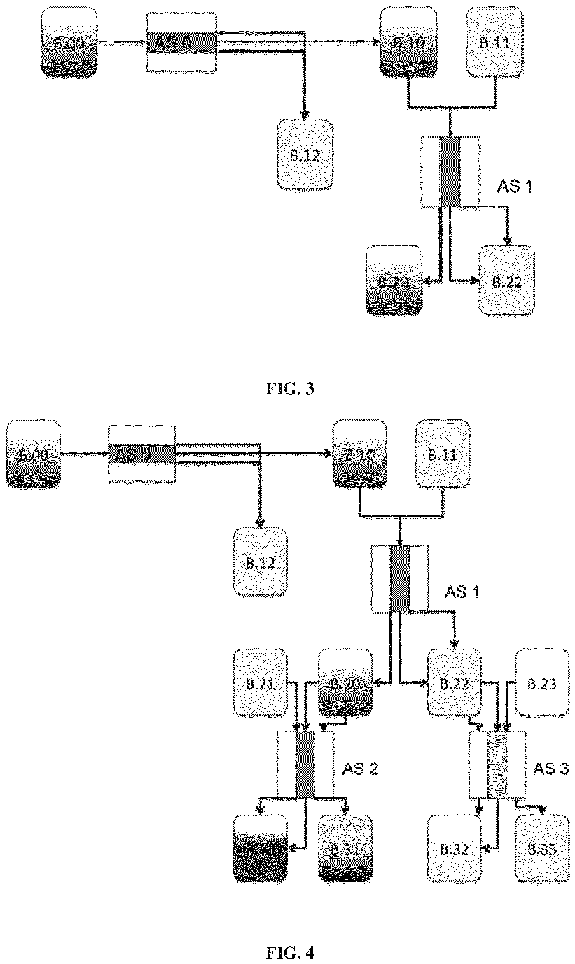

According to one embodiment, as depicted in figure 3, the closed disposable sterile multiple blood bag system further comprises:

- third and a fourth sampling bag **B.20, B.22,** each comprising at least one inlet port and at least one outlet port;
- a second buffer bag **B.11** comprising at least one outlet port; said second buffer bag **B.11** containing a buffer medium;
- second means for transferring fluid **AS1** from the first sampling bag **B.10** to the third and fourth sampling bags **B.20, B.22;** and

wherein the second means for transferring fluid **AS1** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength $\lambda$ within the channel;

wherein the thickness of the channel is equal to $\lambda/2$; the at least one outlet port of the first sampling bag **B.10** is fluidly and sterilely connected to the at least one inlet of the second means for transferring fluid **AS1,** the at least first and second outlets of the second means for transferring fluid **AS1** are fluidly and sterilely connected to the inlet port of respectively the third and fourth sampling bags **B.20, B.22;** and the outlet of the second buffer bag **B.11** is fluidly and sterilely connected to the at least one inlet of the second means for transferring fluid **AS1.**

According to one embodiment, the closed disposable sterile multiple blood bag system further comprises:

- fifth and sixth sampling bag **B.30, B.31,** each comprising at least one inlet port and at least one outlet port;
- a third buffer bag **B.21** comprising at least one outlet port; said third buffer bag **B.21** containing a buffer medium;

- third means for transferring fluid **AS2** from the third sampling bag **B.20** to the fifth and sixth sampling bags **B.30, B.31;** and wherein

the third means for transferring fluid **AS2** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength λ within the channel;

wherein the thickness of the channel is equal to λ/2; the at least one outlet port of the third sampling bag **B.20** is fluidly and sterilely connected to the at least one inlet of the third means for transferring fluid **AS2,** the at least first and second outlets of the third means for transferring fluid **AS2** are fluidly and sterilely connected to the inlet port of respectively the fifth and sixth sampling bags **B.30, B.31;** and the outlet of the third buffer bag **B.21** is fluidly and sterilely connected to the at least one inlet of the third means for transferring fluid **AS2.**

According to one embodiment as depicted in figure 4, the closed disposable sterile multiple blood bag system further comprises:

- seventh and eight sampling bag **B.32, B.33,** each comprising at least one inlet port and at least one outlet port;
- a fourth buffer bag **B.23** comprising at least one outlet port; said fourth buffer bag containing a buffer medium;
- fourth means for transferring fluid **AS3** from the fourth sampling bag **B.22** to the seventh and eight sampling bag **B.32, B.33;** and wherein

the fourth means for transferring fluid **AS3** comprises:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);

- at least one inlet in fluid communication with the channel;
- at least first and second outlets in fluid communication with the channel, the first outlet being separated, on the second transverse axis (z), from the second outlet; and
- at least one acoustic wave generator for generating acoustic wave having a wavelength λ within the channel;

wherein the thickness of the channel is equal to λ/2; the at least one outlet port of the fourth sampling bag **B.22** is fluidly and sterilely connected to the at least one inlet of the fourth means for transferring fluid **AS3,** the at least first and second outlets of the fourth means for transferring fluid **AS3** are fluidly and sterilely connected to the inlet port of respectively the seventh and eight sampling bag **B.32, B.33;** the outlet of the fourth buffer bag **B.23** is fluidly and sterilely connected to the at least one inlet of the fourth means for transferring fluid **AS3.**

According to one embodiment, the fluid collecting bag **B.00** has a volume ranging from 250mL to 1L, preferably from 350 to 530mL.

According to one embodiment, the second sampling bag **B.12** has a volume ranging from 200mL to 750mL, preferably about 280mL.

According to one embodiment, the initial sampling bag has a volume of about 30mL.

According to one embodiment, the seventh sampling bag **B.32** has a volume ranging from 20mL to 100mL, preferably about 50mL.

According to one embodiment, the third and/or fifth sampling bag **B.20, B.30** have a volume ranging from 200mL to 750mL, preferably about 280mL.

According to one embodiment, the buffer medium is an additive solution for preservation and/or for anticoagulation. According to one exemplary embodiment, the additive solution for preservation is selected from SAG-Mannitol (SAGM), PSA IIIm or SSP+.

According to one exemplary embodiment, the additive solution for anticoagulation is a citrate-phosphate-dextrose solution (CPD).

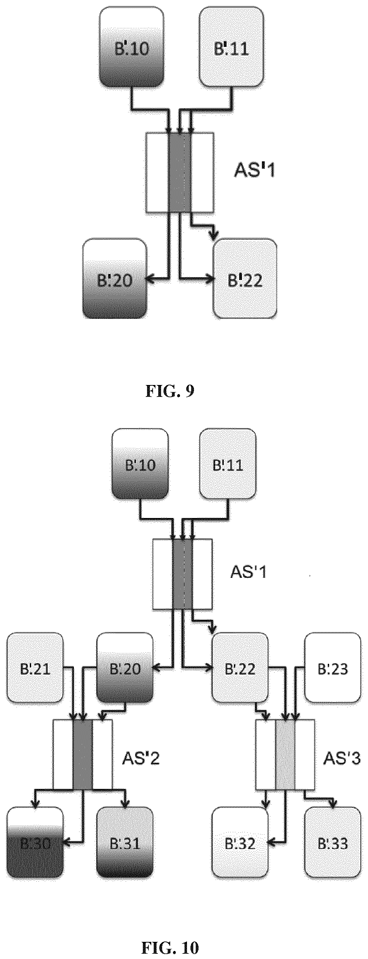

According to one embodiment, as depicted in figure 9, the system comprises a fluid collecting bag **B'.10,** at least first and second sampling bags **B'.20, B'.22,** each bag **B'.10, B'.20, B'.22** comprising at least one inlet port and at least one outlet port; means for transferring fluid **AS'1** from the collecting bag **B'.10** to the sampling bags **B'.20, B'.22,** and a first bag comprising a buffer medium **B'.11** comprising at least one outlet port.

Said means for transferring fluid **AS'1,** also called acoustic sorter, comprise:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);

- at least first and second inlets in fluid communication with the channel, the first inlet being separated (i.e. spaced and distinct), on the second transverse axis (z), from the second inlet; and
- at least first and second outlets in fluid communication with the channel, the first outlet being separated (i.e. spaced and distinct), on the second transverse axis (z), from the second outlet.

According to one embodiment, the means for transferring fluid **AS'1** comprises a single inlet in fluid communication with the channel.

According to one embodiment, the means for transferring fluid **AS'1** further comprises at least one acoustic wave generator for generating acoustic wave having a wavelength λ within the channel. In said embodiment, the thickness of the channel is equal to λ/2.

By applying an acoustic force field over the thickness and over the width of the channel, by means of an acoustic wave generator, it may be possible to move a set of particles depending of their sizes in any area of the channel, and thus to sort and fractionate a biological fluid.

As depicted in figure 9, the at least one outlet port of the collecting bag **B'.10** is fluidly and sterilely connected to at least one of the inlet of the means for transferring fluid **AS'1;** the at least first and second outlets of the means for transferring fluid **AS'1** are fluidly and sterilely connected to the at least one inlet port of respectively the first and second sampling bags **B'.20, B'.22;** and the at least one outlet port of the first bag comprising a buffer medium **B'.11** is fluidly and sterilely connected to at least one of the inlets of the means for transferring fluid **AS'1.**

According to one embodiment, the at least one outlet port of the collecting bag **B'.10** is fluidly and sterilely connected to the at least one outlet port of the first bag comprising a buffer medium **B'.11** upstream of the single inlet of the means for transferring fluid **AS'1.**

According to one embodiment, the fluid connections between the transferring means and the bags comprise any means known by one skilled in the art, such as flexible manifolds or tubes and clamps or valves. Their representations in the drawings are not representative in dimensions and positions.

According to one embodiment, the biological fluid is whole blood and the multiple bag system allows fractionating of blood products such as RBC, WBC, PC and blood plasma, such as PPP or PRP, without centrifugation.

According to one embodiment, the fluid collecting bag **B'.10** is a blood collecting bag for collecting the whole blood, the first sampling bag **B'.20** is a red blood cell, white blood cell and buffer storing bag and the second sampling bag **B'.22** is a plasma storing bag such as a platelet rich plasma storing bag. According to one embodiment, the fluid collecting bag **B'.10** has a volume ranging from 500mL to 1L, preferably about 700mL.

According to another embodiment, the multiple bag system of the invention is used to separate the plasma from the blood cells (plasmapheresis). According to said embodiment, the fluid collecting bag **B'.10** is a blood collecting bag for collecting the whole blood, the first sampling bag **B'.20** is a blood cell storing bag and the second sampling bag **B'.22** is a blood plasma storing bag.

According to one embodiment, the whole blood can be a whole blood derivate. According to one embodiment, the whole blood derivate can be a platelet rich plasma (in order to separate plasma from platelets or concentrate platelets), a buffy coat (in order to isolate platelets form the red blood cells and white blood cells), or plasma (in order to operate plasma washing).

According to one embodiment, the inlet port of the blood collecting bag **B'.10** is fluidly and sterilely connected to a vein puncture needle.

According to one embodiment, the multiple bag system further comprises a third and a fourth sampling bag **B'.30, B'.31,** each comprising at least one inlet port and at least one outlet port; second means for transferring fluid **AS'2** from the first sampling bag **B'.20** to the third and fourth sampling bags **B'.30, B'.31** and a second bag comprising a buffer medium **B'.21** comprising at least one outlet port. Within said extended system, the at least one outlet port of the first sampling bag **B'.20** is fluidly and sterilely connected to at least one of the inlets of the second means for transferring fluid **AS'2** the at least first and second outlets of the second means for transferring fluid **AS'2** are fluidly and sterilely connected to the inlet port of respectively the third and fourth sampling bags **B'.30, B'.31;** and the outlet of the second bag comprising a buffer medium **B'.21** is fluidly and sterilely connected to at least one of the inlets of the second means for transferring fluid **AS'2.**

According to one embodiment, the at least one outlet port of the first sampling bag **B'.20** is fluidly and sterilely connected to the at least one outlet port of the second bag comprising a buffer medium **B'.21** upstream of the single inlet of the second means for transferring fluid **AS'2.**

According to one embodiment, the multiple bag system further comprises a fifth and a sixth sampling bag **B'.32, B'.33,** each comprising at least one inlet port and at least one outlet port; third means for transferring fluid **AS'3** from the second sampling bag **B'.22** to the fifth and sixth sampling bags **B'.32, B'.33,** and a third bag comprising a buffer medium **B'.23** comprising at least one outlet port. Within said extended system, the at least one outlet port of the second sampling bag **B'.22** is fluidly and sterilely connected to at least one of the inlets of the third means for transferring fluid **AS'3,** the at least first and second outlets of the third means for transferring fluid **AS'3** are fluidly and sterilely connected to the inlet port of respectively the fifth and sixth sampling bags **B'.32, B'.33;** and the outlet of the third bag comprising a buffer medium **B'.23** is fluidly and sterilely connected to at least one of the inlets of the third means for transferring fluid **AS'3.**

According to one embodiment, the at least one outlet port of the first sampling bag **B'.22** is fluidly and sterilely connected to the at least one outlet port of the third bag comprising a buffer medium **B'.23** upstream of the single inlet of the third means for transferring fluid **AS'3.**

According to one embodiment, as depicted in figure 10, the multiple bag system further comprises a third, a fourth, a fifth and a sixth sampling bag **B'.30, B'.31, B'.32, B'.33,** each comprising at least one inlet port and at least one outlet port; second means for transferring fluid **AS'2** from the first sampling bag **B'.20** to the third and fourth sampling bags **B'.30, B'.31,** third means for transferring fluid **AS'3** from the second sampling bag **B'.22** to the fifth and sixth sampling bags **B'.32, B'.33,** and a second and a third bag comprising a buffer medium **B'.21, B'.23,** each comprising at least one outlet port. Within said extended system, the at least one outlet port of the first sampling bag **B'.20** is fluidly and sterilely connected to at least one of the inlets of the second means for transferring fluid **AS'2,** the at least first and second outlets of the second means for transferring fluid **AS'2** are fluidly and sterilely connected to the inlet port of respectively the third and fourth sampling bags **B'.30, B'.31,** the outlet of the second bag comprising a buffer medium **B'.21** is fluidly and sterilely connected to at least one of the inlets of the second means for transferring fluid **AS'2,** the at least one outlet port of the second sampling bag **B'.22** is fluidly and sterilely connected to at least one of the inlets of the third means for transferring fluid **AS'3,** the at least first and second outlets of the third means for transferring fluid **AS'3** are fluidly and sterilely connected to the inlet port of respectively the fifth and sixth sampling bags **B'.32, B'.33;** and the outlet of the third bag comprising a buffer medium **B'.23** is fluidly and sterilely connected to at least one of the inlets of the third means for transferring fluid **AS'3.**

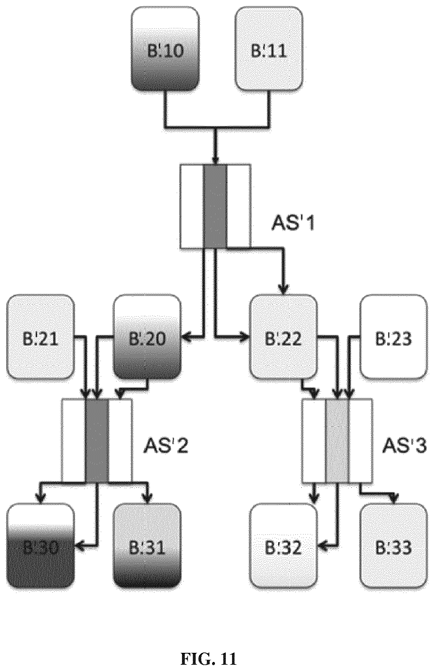

According to one embodiment as depicted in figure 11, the means for transferring fluid **AS'1** comprises a single inlet in fluid communication with the channel and the at least one outlet port of the collecting bag **B'.10** is fluidly and sterilely connected to the at least one outlet port of the first bag comprising a buffer medium **B'.11** upstream of the single inlet of the means for transferring fluid **AS'1.**

According to one embodiment, the third sampling bag **B'.30** is a red blood cell storing bag and the fourth sampling bag **B'.31** is a white blood cell storing bag. According to one embodiment, the fifth sampling bag **B'.32** is a plasma storing bag and the sixth sampling bag **B'.33** is a platelet concentrated storing bag.

According to one embodiment, the first, second and third bags comprising a buffer medium **B'.11, B'.21, B'.23** comprise any buffer medium known by one skilled in the art. Especially, **B'.11** and **B'.21** may comprise anticoagulant such as for instance citrate-phosphate-dextrose solution (CPD) and/or additive solution for preservation such as SAG-Mannitol (SAGM), PSA IIIm or SSP+; and **B'.23** may comprise a preservative medium, such as for instance SAG-Mannitol (SAGM), PSA IIIm or SSP+PAS IIIm.

According to one embodiment, the second and third means for transferring fluid **AS'2, AS'3** comprise:

- a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);

- at least first and second inlets in fluid communication with the channel, the first inlet being separated, on the second transverse axis (z), from the second inlet; and

- at least first and second outlets in fluid communication with the channel, the first outlet being separated, on the second transverse axis (z), from the second outlet.

According to one embodiment, the means for transferring fluid **AS0, AS1, AS2, AS3, AS'1, AS'2, AS'3** (also referred to as acoustic sorters) are identical. According to one embodiment, the means for transferring fluid **AS0, AS1, AS2, AS3, AS'1, AS'2, AS'3** are not identical.

According to one embodiment, the means for transferring fluid **AS0, AS1, AS2, AS3, AS'1, AS'2, AS'3** comprises at least first and second inlets in fluid communication with the channel, the first inlet being separated, on the second transverse axis (z), from the second inlet.

According to one embodiment, one or more of the means for transferring fluid **AS0, AS1, AS2, AS3, AS'1, AS'2, AS'3** further comprises at least a first transverse separation wall separating the first and second inlets.

According to one embodiment, at least one of the inlets of the means for transferring fluid **AS0, AS1, AS2, AS3, AS'1, AS'2, AS'3** has a width equal to the width of the channel. According to one embodiment, one or more of the means for transferring fluid **AS0, AS1, AS2, AS3, AS'1, AS'2, AS'3** further comprises at least a third inlet in fluid communication with the channel, the second inlet being disposed on the second transverse axis (z) between the first and third inlets.

According to one embodiment, one or more of the means for transferring fluid **AS0, AS1, AS2, AS3, AS'1, AS'2, AS'3** further comprises at least a third outlet in fluid communication with the channel, the second outlet being disposed on the second transverse axis (z) between the first and third outlets.

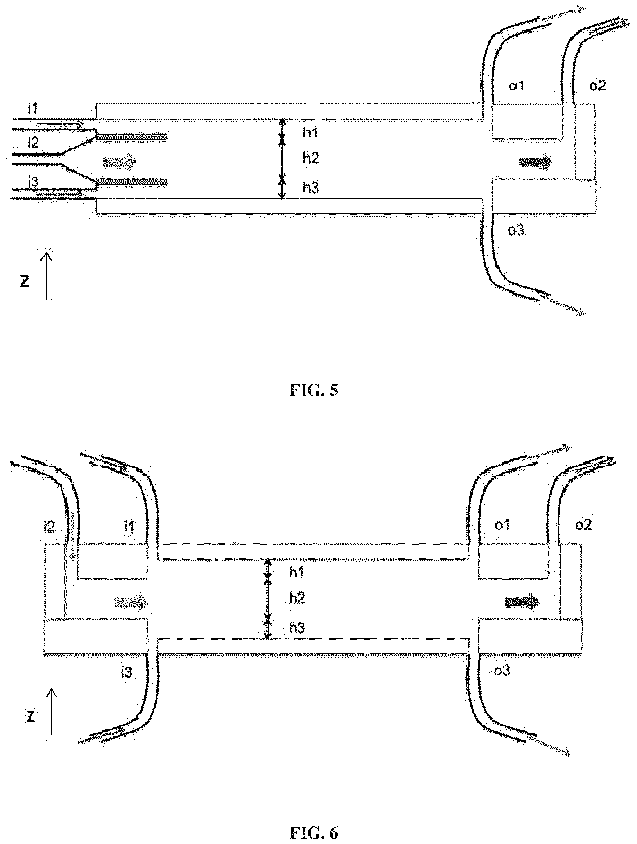

According to one embodiment wherein the means for transferring fluid **AS0, AS1, AS2, AS3, AS'1, AS'2, AS'3** comprises at least three inlets, one or more of the means for transferring fluid **AS0, AS1, AS2, AS3, AS'1, AS'2, AS'3** further comprises first and second transverse separation walls respectively separating the first and second inlets and the second and third inlets, the first and second separation walls being arranged in such a manner that the second inlet is separated from each of said bottom and top walls by a non-zero distance measured along the second transverse axis (z). Said embodiment, depicted in figure 5, enables decoupling the second inlet from the first and third inlets.

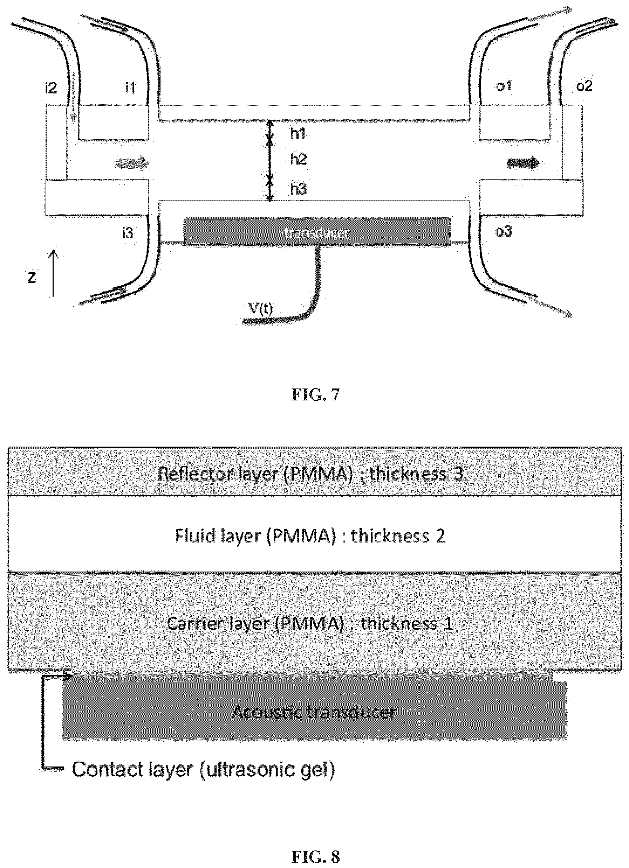

According to one embodiment, as depicted in figures 6 and 7, the first, second and third inlets **i1, i2, i3** of one or more of the means for transferring fluid **AS0, AS1, AS2, AS3, AS'1, AS'2, AS'3** open out in the first or second walls of the channel perpendicularly to the longitudinal axis. According to one embodiment, as depicted in figures 6 and 7, the first and third inlets **i1, i3** of one or more of the means for transferring fluid **AS0, AS1, AS2, AS3, AS'1, AS'2, AS'3** open out respectively in the top and bottom walls of the channel, perpendicularly to the longitudinal axis and facing each other.

According to one embodiment, as depicted in figure 5, the first, second and third inlets **i1, i2, i3** of one or more of the means for transferring fluid **AS0, AS1, AS2, AS3, AS'1, AS'2, AS'3** open out in the channel parallel to the longitudinal axis.

According to one embodiment, the inlets and the outlets of the means for transferring fluid **AS0, AS1, AS2, AS3, AS'1, AS'2, AS'3** are symmetrical.

According to one embodiment, each inlet creates its own fluid layer in the channel and the fluid layers do not mix unless acoustic waves are applied. The use of acoustic force fields for handling object is described for instance in US patent application US 2014/0230912. By applying an acoustic force field over the thickness and over the width, it may be possible to move a set of particles depending of their sizes in any area of the channel, and thus to sort and fractionate biological fluid. According to one embodiment, as depicted in figures 5 to 7, the upper and lower fluid layers **h1, h3** within the channel have a height ranging from 0.05mm to 0.3mm and the middle fluid layer **h2** has a height ranging from 0.1mm to 0.8mm.

According to one embodiment wherein the first means for transferring fluid **AS0** comprises at least three inlets **i1, i2, i3,** the at least one outlet port of the fluid collecting bag **B.00** is fluidly and sterilely connected to the first and third inlets **i1, i3** of the first means for transferring fluid **AS0** and the at least one outlet port of the first buffer bag comprising a buffer medium is fluidly and sterilely connected to the second inlet **i2** of the first means for transferring fluid **AS0**.

According to one embodiment wherein the first means for transferring fluid **AS0** comprises at least three outlets **o1, o2, o3,** the second outlet **o2** of the first means for transferring fluid **AS0** is fluidly and sterilely connected to the inlet port of the first sampling bag **B.10** and the first and third outlets **o1, o3** of the first means for transferring fluid **AS0** are fluidly and sterilely connected to the inlet port of the second sampling bag **B.12**.

According to one embodiment wherein the second means for transferring fluid **AS1** comprises at least three inlets **i1, i2, i3**, the at least one outlet port of the first sampling bag **B.10** is fluidly connected to the first and third inlets **i1, i3** of the second means for transferring fluid **AS1** and the at least one outlet port of the second buffer bag **B.11** comprising a buffer medium is fluidly connected to the second inlet **i2** of the second means for transferring fluid **AS1**.

According to one embodiment wherein the second means for transferring fluid **AS1** comprises at least three outlets **o1, o2, o3**, the second outlet **o2** of the second means for transferring fluid **AS1** is fluidly and sterilely connected to the inlet port of the fourth sampling bag **B.22** and the first and third outlets **o1, o3** of the second means for transferring fluid **AS1** are fluidly connected to the inlet port of the third sampling bag **B.20**.

According to one embodiment wherein the third means for transferring fluid **AS2** comprises at least three inlets **i1, i2, i3**, the at least one outlet port of the third sampling bag **B.20** is fluidly and sterilely connected to the first and third inlets **i1, i3** of the third means for transferring fluid **AS2** and the at least one outlet port of the third buffer bag **B.21** comprising a buffer medium is fluidly and sterilely connected to the second inlet **i2** of the third means for transferring fluid **AS2**.

According to one embodiment wherein the third means for transferring fluid **AS2** comprises at least three outlets **o1, o2, o3,** the second outlet **o2** of the third means for transferring fluid **AS2** is fluidly and sterilely connected to the inlet port of the sixth sampling bag **B.31** and the first and third outlets **o1, o3** of the third means for transferring fluid **AS2** are fluidly and sterilely connected to the inlet port of the fifth sampling bag **B.30**.

According to one embodiment wherein the fourth means for transferring fluid **AS3** comprises at least three inlets **i1, i2, i3**, the at least one outlet port of the fourth sampling bag **B.22** is fluidly and sterilely connected to the first and third inlets **i1, i3** of the fourth means for transferring fluid **AS3** and the at least one outlet port of the fourth buffer bag **B.23** comprising a buffer medium is fluidly and sterilely connected to the second inlet **i2** of the fourth means for transferring fluid **AS3**.

According to one embodiment wherein the fourth means for transferring fluid **AS3** comprises at least three outlets **o1, o2, o3,** the second outlet **o2** of the fourth means for transferring fluid **AS0** is fluidly connected to the inlet port of the first sampling bag **B.10** and the first and third outlets **o1, o3** of the fourth means for transferring fluid **AS3** are fluidly connected to the inlet port of the second sampling bag **B.12**.

According to one embodiment, as depicted in figure 7, the first and second walls of the channel along the second transverse axis (z) are a transmitter or carrier layer and a reflector layer.

According to one embodiment, the reflector layer is made from a metal, preferably titanium or stainless steel.

According to one embodiment, the means for transferring fluid **AS0, AS1, AS2, AS3, AS'1, AS'2, AS'3**, especially the first and second walls, is made from a material chosen among mineral or organic glasses, quartz, thermoplastic materials such as PMMA or polycarbonate, and metals.

According to one embodiment, the channel has a thickness ranging from 0.2mm to 2mm, preferably about 0.750mm, a width ranging from 2mm to 20mm and a length ranging from 10mm to 200mm. According to one embodiment, the channel has a thickness higher than 300 micrometers, preferably ranging from 375 micrometers to 750 micrometers.

According to one embodiment, the channel has a length, measured along the longitudinal axis ranging from 3mm to 20cm, preferably from 3mm to 10cm, more preferably from 10mm to 70mm. According to one embodiment, the channel has a length, measured along the longitudinal axis higher than 10 centimeters.

According to one embodiment, the channel has a width higher than 10 millimeters.

According to one embodiment, the channel has a substantially rectangular cross-section along at least a portion of its length. According to one embodiment, the width/thickness ratio of the channel is greater than 2. According to one embodiment, the length/thickness ratio of the channel is greater than 10.

According to one embodiment, the thickness and the width of the channel are constant along the longitudinal axis. According to one embodiment, the thickness and the width of the channel are variable along the longitudinal axis.

According to one embodiment, the carrier layer has a thickness ranging from 0.2mm to 2mm, preferably about 1mm.

According to one embodiment, the reflector layer has a thickness ranging from 0.2mm to 2mm, preferably about 0.5mm.

As well known to one skilled in the art of acoustophoresis, the thickness of the reflector layer may be half the thickness of the carrier layer or the thickness of the reflector layer may be equal to the thickness of the carrier layer.

According to one embodiment, as shown in figures 7 and 8, one or more of the means for transferring fluid **AS0, AS1, AS2, AS3, AS'1, AS'2, AS'3** further comprise at least one acoustic wave generator which generates acoustic waves in the channel from at least one of the walls. According to one embodiment, one or more of the means for transferring fluid **AS0, AS1, AS2, AS3, AS'1, AS'2, AS'3** comprises a plurality of acoustic wave generators arranges along the channel; said plurality of acoustic wave generators being preferably positioned on the same side of the channel. According to one embodiment, the acoustic waves are generated at a frequency ranging from 0.5MHz to 10MHz, preferably about 1MHz.

According to one embodiment, the at least one acoustic wave generator is configured for generating acoustic wave having a wavelength λ within the channel. In said embodiment, the thickness of the channel is equal to $\frac{\lambda}{2}$, or the thickness of the channel is equal to a multiple of $\frac{\lambda}{2}$. By "the thickness of the channel is equal to a multiple of $\frac{\lambda}{2}$", it should be understood "the thickness of the channel is equal to $n * \frac{\lambda}{2m}$, wherein n and m are integers. According to one embodiment, the thickness of the channel of means for transferring fluid (**AS0, AS1, AS2, AS3, AS'1, AS'2, AS'3**) is equal to λ/2, to λ/3, to λ/4, to λ/5, to λ/6 to λ/8, or to λ/10.

According to one embodiment, the acoustic wave generator is configured for generating volumetric acoustic standing waves. According to one embodiment, the acoustic wave generator is not configured for generating surface acoustic waves.

According to one embodiment, as depicted in figure 8, the acoustic wave generator or transducer is pressed on one of the walls of the means for transferring fluid **AS0, AS1, AS2, AS3, AS'1, AS'2, AS'3**. According to said embodiment, a proper medium such as for instance ultrasonic gel is positioned between the transducer and the wall to ensure good transmission of the acoustic waves.

According to one alternative embodiment, as depicted in figure 7, the acoustic wave generator or transducer is integrated within one of the walls, such as for instance by bonding or any other means known by one skilled in the art.

According to one embodiment, the at least one acoustic wave generator is coupled to the first wall by a dry acoustic coupling. In said embodiment, the first wall is the transmitter or carrier layer and the second wall is the reflector layer.

According to one embodiment, the at least one acoustic wave generator is coupled to the first wall (i.e. the transmitter or carrier layer) with a coupling layer.

According to one embodiment, said coupling layer is made from thermoplastic elastomers, thermoplastic polyurethanes or silicone. According to one embodiment, said coupling layer has a hardness ranging from 5 to 50 Shore A. According to one embodiment, the attenuation within the coupling layer is ranging from 0 to 1 dB/mm. According to one embodiment, the attenuation at the dry interface is lower than 8dB (compared to the attenuation with a gel acoustic coupling). According to one embodiment, the contact pressure at the dry interface is ranging from 12 to 60 kPa.

According to one exemplary embodiment, the coupling layer is made from Aqualene® commercialized by OLYMPUS. According to one embodiment, said coupling layer is made of oil or a mixture comprising oil.

According to one embodiment, the acoustic conductance coefficient of the first wall (i.e. the transmitter or carrier layer) is ranging from 0.5 to 1, preferably from 0.75 to 1, more preferably from 0.9 to 1. According to one embodiment, the first wall (i.e. the transmitter or carrier layer) is made from a material exhibiting an acoustic conductance coefficient ranging from 0.5 to 1, preferably from 0.75 to 1, more preferably from 0.9 to 1.

According to one embodiment, the acoustic reflection coefficient of the second wall (i.e. the reflector layer) is ranging from 0.5 to 1, preferably from 0.75 to 1, more preferably from 0.9 to 1. According to one embodiment, the second wall (i.e. the reflector layer) is made from a material exhibiting an acoustic reflection coefficient ranging from 0.5 to 1, preferably from 0.75 to 1, more preferably from 0.9 to 1.

According to one embodiment, the closed disposable sterile multiple blood bag system further comprises at least one bag with additives solution.

According to one embodiment, the closed disposable sterile multiple blood bag system does not comprise active flow system, such as pumps or flow restrictors.

According to one embodiment, the closed disposable sterile multiple blood bag system does not comprise electrical connections.

According to one embodiment, the closed disposable sterile multiple blood bag system does not comprise a piezoelectric substrate.

According to one embodiment, the closed disposable sterile multiple blood bag system is not a washing system.

According to one embodiment, the channels of the means for transferring fluid are acoustic resonators. According to one embodiment, the channels of the means for transferring fluid are not one quarter wave separation chambers.

**BRIEF DESCRIPTION OF THE DRAWINGS**

Figure 1A shows the principles of production of various blood products using centrifugation according to the prior art.

Figure 1B illustrates the stratification of various blood products after centrifugation of whole blood according to the prior art.

Figures 2, 3 and 4 depict a multiple blood bag system according to various embodiments of the invention.

Figures 5 and 6 illustrate side-view of an acoustic sorter according to various embodiments of the invention.

Figure 7 is a side-view of an acoustic sorter according to one embodiment of the invention further comprising an integrated transducer.

Figure 8 depicts the multiples layer of an acoustic sorter according to one embodiment of the invention.

Figures 9, 10 and 11 illustrate a multiple blood bag system according to various embodiments of the present invention.

**Claims**

1. A method for high throughput preparation of blood products to be used for blood transfusion, the method comprising the following steps:

    - providing a closed disposable sterile multiple blood bag system comprising:

        - a fluid collecting bag (B.00) comprising at least one outlet port, said fluid collecting bag containing whole blood obtained from an individual;
        - first and second sampling bags (B.10, B.12), each comprising at least one inlet port and at least one outlet port;
        - a second buffer bag (B.11) comprising at least one outlet port; said second buffer bag (B.11) containing a buffer medium;
        - third and fourth sampling bags (B.20, B.22), each comprising at least one inlet port and at least one outlet port;
        - first means for transferring fluid (AS0) from the first collecting bag (B.00) to the sampling bags (B.10, B.12), and second means for transferring fluid (AS1) from the first sampling bag (B.10) to the third and fourth sampling bags (B.20, B.22), wherein the first and second means for transferring fluid (ASO, AS1) each comprise:

            • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
            • at least one inlet in fluid communication with the channel;

• at least first and second outlets in fluid communication with the channel, the first outlet being separated, on the second transverse axis (z), from the second outlet; and

the at least one outlet port of the fluid collecting bag (B.00) is sterilely and fluidly connected to the at least one inlet of the first means for transferring fluid (AS0) and the at least first and second outlets of the first means for transferring fluid (AS0) are sterilely and fluidly connected to the at least one inlet port of respectively the first and second sampling bags (B.12, B.10); the at least one outlet port of the first sampling bag (B.10) is fluidly and sterilely connected to the at least one inlet of the second means for transferring fluid (AS 1), the at least first and second outlets of the second means for transferring fluid (AS1) are fluidly and sterilely connected to the inlet port of respectively the third and fourth sampling bags (B.20, B.22), and the at least one outlet port of the second buffer bag (B.11) is fluidly and sterilely connected to the at least one inlet of the second means for transferring fluid (AS1); and

- applying acoustic field inside the channel of the means for transferring fluid (AS0) by means of an acoustic wave generator;
- transferring the content of the fluid collecting bag (B.00) in the first means for transferring fluid (AS0); and
- collecting blood cells in the first sampling bag (B.10) and blood plasma in the second sampling bag (B.12);
- applying acoustic field inside the channel of the second means for transferring fluid (AS1) by means of the acoustic wave generator;
- transferring the content of the first sampling bag (B.10) and the second buffer bag (B.11) in the second means for transferring fluid (AS1); and
- collecting red blood cell concentrate in the third sampling bag (B.20) and platelet concentrate in the fourth collecting bag (B.22).

2. The method for high throughput preparation of blood products according to claim 1, wherein the closed disposable sterile multiple blood bag system further comprises a first buffer bag comprising at least one outlet port, said first buffer bag containing a buffer medium, and said at least one outlet port is sterilely and fluidly connected to the at least one inlet of the first means for transferring fluid (AS0).

3. The method for high throughput preparation of blood products according to claim 1 or claim **2,** wherein

- the closed disposable sterile multiple blood bag system further comprises:

    - a third buffer bag (B.21) comprising at least one outlet port; said third buffer bag (B.21) containing a buffer medium;
    - fifth and sixth sampling bags (B.30, B.31), each comprising at least one inlet port and at least one outlet port;
    - third means for transferring fluid (AS2) from the third sampling bag (B.20) to the fifth and sixth sampling bags (B.30, B.31), wherein the third means for transferring fluid (AS2) comprises:

        • a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
        • at least one inlet in fluid communication with the channel;
        • at least first and second outlets in fluid communication with the channel, the first outlet being separated, on the second transverse axis (z), from the second outlet; and

    the at least one outlet port of the third sampling bag (B.20) is fluidly and sterilely connected to the at least one inlet of the third means for transferring fluid (AS2), the at least first and second outlets of the third means for transferring fluid (AS2) are fluidly and sterilely connected to the inlet port of respectively the fifth and sixth sampling bags (B.30, B.31), and the outlet of the third buffer bag is fluidly and sterilely connected to the at least one inlet of the third means for transferring fluid (AS2); and

wherein the method further comprises the following steps:

    - applying acoustic field inside the channel of the third means for transferring fluid (AS2) by means of the acoustic wave generator;
    - transferring the content of the third sampling bag (B.20) and the third buffer bag (B.21) in the third means for transferring fluid (AS2); and

- collecting red blood cell concentrate in the fifth sampling bag (B.30) and white blood cell concentrate in the sixth collecting bag (B.31).

4. The method for high throughput preparation of blood products according to anyone of claims **1** to **3,** wherein

- the closed disposable sterile multiple blood bag system further comprises:

- a fourth buffer bag (B.23) comprising at least one outlet port; said fourth buffer bag (B.23) containing a buffer medium;
- seventh and eight sampling bags (B.32, B.33), each comprising at least one inlet port and at least one outlet port;
- fourth means for transferring fluid (AS3) from the fourth sampling bag (B.22) to the seventh and eight sampling bags (B.32, B.33), wherein the means for transferring fluid (AS3) comprises:

• a channel extending along a longitudinal axis, the channel having a cross section with a width measured along a first transverse axis and a thickness measured along a second transverse axis (z) perpendicular to the first transverse axis, the width being greater than or equal to the thickness, the channel having first and second walls along the second transverse axis (z);
• at least one inlet in fluid communication with the channel;
• at least first and second outlets in fluid communication with the channel, the first outlet being separated, on the second transverse axis (z), from the second outlet; and

the at least one outlet port of the fourth sampling bag (B.22) is fluidly and sterilely connected to the at least one inlet of the fourth means for transferring fluid (AS3), the at least first and second outlets of the fourth means for transferring fluid (AS3) are fluidly and sterilely connected to the inlet port of respectively the seventh and eight sampling bags (B.32, B.33), and the outlet of the fourth buffer bag (B.23) is fluidly and sterilely connected to the at least one inlet of the fourth means for transferring fluid (AS3); and

wherein the method further comprises the following steps:

- applying acoustic field inside the channel of the fourth means for transferring fluid (AS3) by means of the acoustic wave generator;
- transferring the content of the fourth sampling bag (B.22) and the fourth buffer bag (B.23) in the fourth means for transferring fluid (AS3); and
- collecting platelet concentrate in the seventh sampling bag (B.32).

5. The method for high throughput preparation of blood products according to anyone of claims **1** to **4,** wherein means for transferring fluid further comprises at least one acoustic wave generator for generating acoustic wave having a wavelength $\lambda$ within the channel; and wherein the thickness of the channel of means for transferring fluid is equal to $\frac{\lambda}{2}$ or is

equal to a multiple of $\frac{\lambda}{2}$.

6. The method for high throughput preparation of blood products according to anyone of claims **1** to **5,** wherein the acoustic field generated inside the channel of the means for transferring fluid (ASO, AS1, AS2, AS3) contains volumetric acoustic standing waves.

7. The method for high throughput preparation of blood products according to anyone of claims **1** to **6,** wherein the throughput is ranging from 0.5mL/min to 100mL/min, preferably from 0.5mL/min to 20mL/min.

8. The method for high throughput preparation of blood products according to anyone of claims **1** to **7,** wherein the width/thickness ratio of the channel is greater than 2 or wherein the length/thickness ratio of the channel is greater than 10.

9. The method for high throughput preparation of blood products according to anyone of claims **1** to **8,** wherein the thickness of the channel is higher than 300 micrometers, preferably ranging from 375 micrometers to 750 micrometers.

10. The method for high throughput preparation of blood products according to anyone of claims **1** to **9,** wherein the width of the channel is higher than 10 millimeters.

11. The method for high throughput preparation of blood products according to anyone of claims **1** to **10,** wherein the length of the channel along the longitudinal axis is higher than 10 centimeters.

12. The method for high throughput preparation of blood products according to anyone of claims **1** to **11,** wherein the fluid collecting bag (B.00) comprises an inlet port which is fluidly and sterilely connected to a vein puncture needle.

13. The method for high throughput preparation of blood products according to anyone of claims **1** to **12,** wherein the steps of applying acoustic field inside the channel comprises the step of coupling the acoustic wave generator to the first wall of the channel, and wherein the acoustic conductance of the first wall is ranging from 0.5 to 1.

14. The method for high throughput preparation of blood products according to anyone of claims **1** to **13,** wherein the second wall of the channel is a reflector, and wherein the acoustic reflection coefficient of the second wall is ranging 5 from 0.5 to 1.

**Patentansprüche**

1. Verfahren zur Vorbereitung mit hohem Durchsatz von für Bluttransfusionen zu verwendenden Blutprodukten, wobei das Verfahren die folgenden Schritte umfasst:

- Bereitstellen eines geschlossenen abnehmbaren sterilen Systems mehrerer Blutbeutel, Folgendes umfassend:

- einen Fluidsammelbeutel (B.00), umfassend wenigstens eine Auslassöffnung, wobei der Fluidsammelbeutel von einem Individuum gewonnenes Vollblut enthält;
- erste und zweite Probenahmebeutel (B.10, B.12), jeder wenigstens eine Einlassöffnung und wenigstens eine Auslassöffnung umfassend;
- einen zweiten Pufferbeutel (B.11), wenigstens eine Auslassöffnung umfassend; wobei der zweite Pufferbeutel (B.11) ein Puffermedium umfasst;
- dritte und vierte Probenahmebeutel (B.20, B.22), jeder wenigstens eine Einlassöffnung und eine Auslassöffnung umfassend;
- erste Mittel zum Übertragen von Fluid (AS0) von dem ersten Sammelbeutel (B.00) zu den Probenahmebeuteln (B.10, B.12), und zweite Mittel zum Übertragen von Fluid (AS1) vom ersten Probenahmebeutel (B.10) zu den dritten und vierten Probenahmebeuteln (B.20, B.22), wobei die ersten und zweiten Mittel zum Übertragen von Fluid (ASO, AS1) jeweils Folgendes umfassen:

• einen Kanal, sich entlang einer Längsachse erstreckend, wobei der Kanal einen Querschnitt aufweist mit einer Breite gemessen entlang einer ersten Querachse und einer Dicke gemessen entlang einer zweiten Querachse (z) senkrecht zu der ersten Querachse, wobei die Breite größer als oder gleich wie die Dicke ist, wobei der Kanal erste und zweite Wände entlang der zweiten Querachse (z) aufweist;
• wenigstens einen Einlass in fluidischer Kommunikation mit dem Kanal;
• wenigstens erste und zweite Auslässe in fluidischer Kommunikation mit dem Kanal, wobei der erste Auslass auf der zweiten Querachse (z) von dem zweiten Auslass getrennt ist, und

die wenigstens eine Auslassöffnung des Fluidsammelbeutels (B.00) steril und fluidisch verbunden ist mit dem wenigstens einen Einlass der ersten Mittel zum Übertragen von Fluid (AS0) und die wenigstens ersten und zweiten Auslässe der ersten Mittel zum Übertragen von Fluid (AS0) steril und fluidisch verbunden sind mit der wenigstens einen Einlassöffnung von jeweils den ersten und zweiten Probenahmebeuteln (B.12, B.10); wobei die wenigstens eine Auslassöffnung des ersten Probenahmebeutels (B.10) fluidisch und steril verbunden ist mit dem wenigstens einen Einlass der zweiten Mittel zum Übertragen von Fluid (AS1), die wenigstens ersten und zweiten Auslässe der zweiten Mittel zum Übertragen von Fluid (AS1) fluidisch und steril verbunden sind mit der Einlassöffnung von jeweils den dritten und vierten Probenahmebeuteln (B.20, B.22), und die wenigstens eine Auslassöffnung des zweiten Pufferbeutels (B.11) fluidisch und steril verbunden ist mit wenigstens einem Einlass der zweiten Mittel zum Übertragen von Fluid (AS1); und

- Anwenden eines akustischen Feldes innerhalb des Kanals der Mittel zum Übertragen von Fluid (AS0) anhand eines Schallwellengenerators;
- Übertragen des Inhalts des Fluidsammelbeutels (B.00) in den ersten Mitteln zum Übertragen von Fluid (AS0); und
- Sammeln von Blutzellen in dem ersten Probenahmebeutel (B.10) und von Blutplasma in dem zweiten Probenahmebeutel (B.12);
- Anwenden eines akustischen Feldes innerhalb des Kanals der zweiten Mittel zum Übertragen von Fluid (AS1) anhand des Schallwellengenerators;
- Übertragen des Inhalts des ersten Probenahmebeutels (B.10) und des zweiten Pufferbeutels (B.11) in den zweiten Mitteln zum Übertragen von Fluid (AS1); und
- Sammeln von rotem Blutzellenkonzentrat in dem dritten Probenahmebeutel (B.20) und von Thrombozytenkonzentrat in dem vierten Probenahmebeutel (B.22).

2. Verfahren zur Vorbereitung mit hohem Durchsatz von Blutprodukten nach Anspruch **1,** wobei das geschlossene abnehmbare sterile System mehrerer Blutbeutel weiter einen ersten Pufferbeutel umfasst, umfassend wenigstens eine Auslassöffnung, wobei der erste Pufferbeutel ein Puffermedium umfasst, und die wenigstens eine Auslassöffnung steril und fluidisch verbunden ist mit dem wenigstens einen Einlass der ersten Mittel zum Übertragen von Fluid (AS0).

3. Verfahren zur Vorbereitung mit hohem Durchsatz von Blutprodukten nach Anspruch **1** oder Anspruch **2,** wobei

- das geschlossene abnehmbare sterile System mehrerer Blutbeutel weiter Folgendes umfasst:

- einen dritten Pufferbeutel (B.21), umfassend wenigstens eine Auslassöffnung; wobei der dritte Pufferbeutel (B.21) ein Puffermedium umfasst;
- fünfte und sechste Probenahmebeutel (B.30 B.31), jeweils wenigstens eine Einlassöffnung und wenigstens eine Auslassöffnung umfassend;
- dritte Mittel zum Übertragen von Fluid (AS2) von dem dritten Probenahmebeutel (B.20) zu den fünften und sechsten Probenahmebeuteln (B.30, B.31), wobei die dritten Mittel zum Übertragen von Fluid (AS2) Folgendes umfassen:

• einen Kanal, sich entlang einer Längsachse erstreckend, wobei der Kanal einen Querschnitt aufweist mit einer Breite gemessen entlang einer ersten Querachse und einer Dicke gemessen entlang einer zweiten Querachse (z) senkrecht zu der ersten Querachse, wobei die Breite größer als oder gleich wie die Dicke ist, wobei der Kanal erste und zweite Wände entlang der zweiten Querachse (z) aufweist;
• wenigstens einen Einlass in fluidischer Kommunikation mit dem Kanal;
• wenigstens erste und zweite Ausgänge in fluidischer Kommunikation mit dem Kanal, wobei der erste Auslass auf der zweiten Längsachse (z) von dem zweiten Auslass getrennt ist; und

die wenigstens eine Auslassöffnung des dritten Probenahmebeutels (B.20) fluidisch und steril mit dem wenigstens einen Einlass der dritten Mittel zum Übertragen von Fluid (AS2) verbunden ist, wobei die wenigstens ersten und zweiten Auslässe der dritten Mittel zum Übertragen von Fluid (AS2) fluidisch und steril mit der Einlassöffnung von jeweils den fünften und sechsten Probenahmebeuteln (B.30, B.31) verbunden sind, und der Auslass des dritten Pufferbeutels fluidisch und steril mit dem wenigstens einen Einlass der dritten Mittel zum Übertragen von Fluid (AS2) verbunden ist; und

wobei das Verfahren weiter die folgenden Schritte umfasst:

- Anwenden eines akustischen Feldes innerhalb des Kanals der dritten Mittel zum Übertragen von Fluid (AS2) anhand des Schallwellengenerators;
- Übertragen des Inhalts des dritten Probenahmebeutels (B.20) und des dritten Pufferbeutels (B.21) in den dritten Mitteln zum Übertragen von Fluid (AS2); und
- Sammeln von rotem Blutzellenkonzentrat in dem fünften Probenahmebeutel (B.30) und von weißem Blutzellenkonzentrat in dem sechsten Probenahmebeutel (B.31).

4. Verfahren zur Vorbereitung mit hohem Durchsatz von Blutprodukten nach einem der Ansprüche **1** bis **3,** wobei

- das geschlossene abnehmbare sterile System mehrerer Blutbeutel weiter Folgendes umfasst:

- einen vierten Pufferbeutel (B.23), umfassend wenigstens eine Auslassöffnung; wobei der vierte Pufferbeutel (B.23) ein Puffermedium umfasst;
- siebte und achte Probenahmebeutel (B.32, B.33), jeder wenigstens eine Einlassöffnung und wenigstens eine Auslassöffnung umfassend;
- vierte Mittel zum Übertragen von Fluid (AS3) von dem vierten Probenahmebeutel (B.22) zu den siebten und achten Probenahmebeuteln (B.32, B.33), wobei die Mittel zum Übertragen von Fluid (AS3) Folgendes umfassen:

• einen Kanal, sich entlang einer Längsachse erstreckend, wobei der Kanal einen Querschnitt aufweist mit einer Breite gemessen entlang einer ersten Querachse und einer Dicke gemessen entlang einer zweiten Querachse (z) senkrecht zu der ersten Querachse, wobei die Breite größer als oder gleich wie die Dicke ist, wobei der Kanal erste und zweite Wände entlang der zweiten Querachse (z) aufweist;
- wenigstens einen Einlass in fluidischer Kommunikation mit dem Kanal;
- wenigstens erste und zweite Ausgänge in fluidischer Kommunikation mit dem Kanal, wobei der erste Auslass auf der zweiten Querachse (z) von dem zweiten Auslass getrennt ist; und

die wenigstens eine Auslassöffnung des vierten Probenahmebeutels (B.22) fluidisch und steril verbunden ist mit dem wenigstens einen Einlass der vierten Mittel zum Übertragen von Fluid (AS3), wobei die wenigstens ersten und zweiten Auslässe der vierten Mittel zum Übertragen von Fluid (AS3) fluidisch und steril verbunden sind mit der Einlassöffnung von jeweils den siebten und achten Probenahmebeuteln (B.32, B.33), und der Auslass des vierten Pufferbeutels (B.23) fluidisch und steril verbunden ist mit dem wenigstens einen Einlass der vierten Mittel zum Übertragen von Fluid (AS3); und

wobei das Verfahren weiter die folgenden Schritte umfasst:

- Anwenden eines akustischen Feldes innerhalb des Kanals der vierten Mittel zum Übertragen von Fluid (AS3) anhand des Schallwellengenerators;
- Übertragen des Inhalts des vierten Probenahmebeutels (B.22) und des vierten Pufferbeutels (B.23) in den vierten Mitteln zum Übertragen von Fluid (AS3); und
- Sammeln von Thrombozytenkonzentrat in dem siebten Probenahmebeutel (B.32).

**5.** Verfahren zur Vorbereitung mit hohem Durchsatz von Blutprodukten nach einem der Ansprüche **1** bis **4,** wobei Mittel zum Übertragen von Fluid weiter wenigstens einen Schallwellengenerator zum Generieren von Schallwellen mit einer Wellenlänge λ innerhalb des Kanals umfassen; und wobei die Dicke des Kanals von Mitteln zum Übertragen von Fluid gleich $\frac{\lambda}{2}$ ist oder gleich einem Vielfachen von $\frac{\lambda}{2}$ ist.

**6.** Verfahren zur Vorbereitung mit hohem Durchsatz von Blutprodukten nach einem der Ansprüche **1** bis **5,** wobei das akustische Feld innerhalb des Kanals der Mittel zum Übertragen von Fluid (ASO, AS1, AS2, AS3) volumetrische akustische stehende Wellen enthält.

**7.** Verfahren zur Vorbereitung mit hohem Durchsatz von Blutprodukten nach einem der Ansprüche **1** bis **6,** wobei die Durchsatzrate von 0,5 ml/Min. bis 100 ml/Min. reicht, bevorzugt von 0,5 ml/Min. bis 20 ml/Min.

**8.** Verfahren zur Vorbereitung mit hohem Durchsatz von Blutprodukten nach einem der Ansprüche **1** bis **7,** wobei das Breite-/Dicke-Verhältnis des Kanals größer als 2 ist oder wobei das Breite-/Dicke-Verhältnis des Kanals größer als 10 ist.

**9.** Verfahren zur Vorbereitung mit hohem Durchsatz von Blutprodukten nach einem der Ansprüche **1** bis **8,** wobei die Dicke des Kanals höher ist als 300 Mikrometer, bevorzugt reichend von 375 Mikrometer bis 750 Mikrometer.

**10.** Verfahren zur Vorbereitung mit hohem Durchsatz von Blutprodukten nach einem der Ansprüche **1** bis **9,** wobei die Breite des Kanals größer als 10 Millimeter ist.

**11.** Verfahren zur Vorbereitung mit hohem Durchsatz von Blutprodukten nach einem der Ansprüche **1** bis **10,** wobei die Länge des Kanals entlang der Längsachse höher als 10 Zentimeter ist.

**12.** Verfahren zur Vorbereitung mit hohem Durchsatz von Blutprodukten nach einem der Ansprüche **1** bis **11,** wobei der Fluidsammelbeutel (B.00) eine Einlassöffnung umfasst, die fluidisch und steril mit einer Venenpunktionsnadel verbunden ist.

**13.** Verfahren zur Vorbereitung mit hohem Durchsatz von Blutprodukten nach einem der Ansprüche **1** bis **12,** wobei die Schritte zum Anwenden eines akustischen Feldes innerhalb des Kanals den Schritt des Koppelns des Schallwellengenerators mit der ersten Wand des Kanals umfassen, und wobei die akustische Leitfähigkeit der ersten Wand von 0,5 bis 1 reicht.

**14.** Verfahren zur Vorbereitung mit hohem Durchsatz von Blutprodukten nach einem der Ansprüche **1** bis **13,** wobei die zweite Wand des Kanals ein Reflektor ist, und wobei die Schallreflexion der zweiten Wand von 0,5 bis 1 reicht.

**Revendications**

**1.** Procédé de préparation de produits sanguins à haut débit à utiliser pour transfusion sanguine, le procédé comprenant les étapes suivantes :

- Fournir un système fermé, amovible et stérile de plusieurs poches de sang, comprenant :

- un sac de collecte de fluide (B.00) comprenant au moins une ouverture de sortie, le sac de collecte de fluide contenant un échantillon de sang obtenu à partir d'un individu :
- des premier et deuxième sacs d'échantillonnage (B.10, B.12), comprenant chacun au moins une ouverture d'entrée et au moins une ouverture de sortie :
- un deuxième sac tampon (B.11), comprenant au moins une ouverture de sortie; le deuxième sac tampon (B.11) comprenant un milieu tampon;
- des troisième et quatrième sacs d'échantillonnage (B.20, B.22), comprenant chacun au moins une ouverture d'entrée et une ouverture de sortie :
- des premiers moyens pour transférer le fluide (AS0) du premier sac de collecte (B.00) vers les sacs d'échantillonnage (B.10, B.12), et des seconds moyens pour transférer le fluide (AS1) du premier sac d'échantillonnage (B.10) aux troisième et quatrième sacs d'échantillonnage (B.20, B.22), les premier et deuxième moyens de transfert de fluide (ASO, AS1) comprenant chacun :

     • un canal s'étendant le long d'un axe longitudinal, le canal ayant une section transversale ayant une largeur mesurée le long d'un premier axe transversal et une épaisseur mesurée le long d'un deuxième axe transversal (z) perpendiculaire au premier axe transversal, la largeur étant supérieure ou égale à l'épaisseur, le canal ayant des première et seconde parois le long du deuxième axe transversal (z);
     • au moins une entrée en communication fluidique avec le canal;
     • au moins des première et deuxième sorties en communication fluidique avec le canal, la première sortie sur le deuxième axe transversal (z) étant séparée de la deuxième sortie, et

l'au moins une ouverture de sortie du sac de collecte de fluide (B.00) est connectée de manière stérile et fluide à l'au moins une entrée des premiers moyens de transfert de fluide (AS0) et les au moins première et deuxième sorties des premiers moyens de transfert de fluide (AS0) sont connectés de manière stériles et fluide à l'au moins une ouverture d'entrée de chacun des premier et second sacs d'échantillonnage (B.12, B.10); dans lequel l'au moins une ouverture de sortie du premier sac d'échantillonnage (B.10) est connectée de manière fluidique et stérile à l'au moins une entrée des seconds moyens de transfert de fluide (AS1), les au moins première et seconde sorties des seconds moyens de transfert de fluide (AS1) sont connectés de manière fluidique et stérile à l'ouverture d'entrée des troisième et quatrième sacs d'échantillonnage (B.20, B.22), et l'au moins une ouverture de sortie du deuxième sac tampon (B.11) est connectée de manière fluidique et stérile à au moins une entrée du second moyen pour transférer du fluide (AS1); et

- appliquer un champ acoustique à l'intérieur du canal du moyen de transmission de fluide (AS0) à l'aide d'un générateur d'ondes sonores ;
- transférer le contenu du sac de collecte de fluide (B.00) dans le premier moyen de transfert de fluide (AS0) ; et
- collecter des cellules sanguines dans le premier sac d'échantillonnage (B.10) et le plasma du sang dans le deuxième sac de prélèvement (B.12) ;
- appliquer un champ acoustique à l'intérieur du canal du deuxième moyen de transmission de fluide (AS1) à l'aide du générateur d'ondes sonores ;
- transférer le contenu du premier sac d'échantillonnage (B.10) et du deuxième sac tampon (B.11) dans les seconds moyens de transfert de fluide (AS1) ; et
- collecter un concentré de globules rouges dans le sac d'échantillonnage troisième (B.20) et un concentré de plaquettes dans le quatrième sac de prélèvement (B.22).

2.   Procédé de préparation de produits sanguins à haut débit selon la revendication 1, dans lequel le système stérile amovible fermé de plusieurs poches de sang comprend en outre une première poche tampon comprenant au moins une ouverture de sortie, la première poche tampon comprenant un milieu tampon, et l'au moins une ouverture de sortie est connectée de manière stérile et fluidique à l'au moins une entrée du premier moyen de transfert de fluide (AS0).

3.   Procédé de préparation de produits sanguins à haut débit selon la revendication **1** ou la revendication **2,** dans lequel

     - le système stérile fermé de plusieurs poches de sang amovible comprend en outre :

          - un troisième sac tampon (B.21) comprenant au moins une ouverture de sortie ; le troisième sac tampon (B.21) comprenant un milieu tampon ;
- cinquième et sixième sacs d'échantillonnage (B.30 B.31), comprenant chacun au moins une ouverture d'entrée et au moins une ouverture de sortie ;
- des troisièmes moyens de transfert de fluide (AS2) du troisième sac d'échantillonnage (B.20) aux cinquième et sixième sacs d'échantillonnage (B.30, B.31), les troisièmes moyens de transfert de fluide (AS2) comprenant :

  • un canal s'étendant le long d'un axe longitudinal, le canal ayant une section transversale ayant une largeur mesurée le long d'un premier axe transversal et une épaisseur mesurée le long d'un deuxième axe transversal (z) perpendiculaire au premier axe transversal, la largeur étant supérieure ou égale à l'épaisseur, le canal ayant des première et seconde parois le long du deuxième axe transversal (z);
  • au moins une entrée en communication fluidique avec le canal;
  • au moins des première et seconde sorties en communication fluidique avec le canal, la première sortie sur le deuxième axe longitudinal (z) étant séparée de la deuxième sortie; et

  l'au moins une ouverture de sortie du troisième sac d'échantillonnage (B.20) est connectée de manière fluidique et stérile à l'au moins une entrée du troisième moyen de transfert de fluide (AS2), les au moins première et deuxième sorties du troisième moyen de transfert de fluide (AS2) sont connectés de manière fluidique et stérile à l'ouverture d'entrée des cinquième et sixième sacs d'échantillonnage (B.30, B.31), et la sortie du troisième sac tampon est connectée de manière fluidique et stérile à l'au moins une entrée du troisième moyen de transfert de fluide (AS2); et

dans lequel le procédé comprend en outre les étapes suivantes:

  - appliquer un champ acoustique à l'intérieur du canal du troisième moyen de transmission de fluide (AS2) à l'aide du générateur d'ondes sonores ;
  - transférer le contenu du troisième sac de prélèvement (B.20) et du troisième sac tampon (B.21) dans le troisième moyen de transfert de fluide (AS2) ; et

- Recueillir le concentré de globules rouges dans le cinquième sac de prélèvement (B.30) et le concentré de globules blancs dans le sixième sac de prélèvement (B.31).

4. Procédé de préparation de produits sanguins à haut débit selon l'une quelconque des revendications **1 à 3,** dans lequel

  - le système stérile fermé de plusieurs poches de sang amovible comprend en outre :

    - un quatrième sac tampon (B.23) comprenant au moins une ouverture de sortie ; le quatrième sac tampon (B.23) comprenant un milieu tampon ;
    - septième et huitième sacs d'échantillonnage (B.32, B.33), comprenant chacun au moins une ouverture d'entrée et au moins une ouverture de sortie ;
    - un quatrième moyen pour transférer du fluide (AS3) du quatrième sac de prélèvement (B.22) aux septième et huitième sacs d'échantillonnage (B.32, B.33) dans lequel les moyens de transfert comprennent en fluide (AS3) :

      • un canal s'étendant le long d'un axe longitudinal, le canal ayant une section transversale ayant une largeur mesurée le long d'un premier axe transversal et une épaisseur mesurée le long d'un deuxième axe transversal (z) perpendiculaire au premier axe transversal, la largeur étant supérieure ou égale à l'épaisseur, le canal ayant des première et seconde parois le long du deuxième axe transversal (z);
      • au moins une entrée en communication fluidique avec le canal;
      • au moins des première et seconde sorties en communication fluidique avec le canal, la première sortie sur le deuxième axe transversal (z) étant séparée de la deuxième sortie; et

      l'au moins une ouverture de sortie du quatrième sac d'échantillonnage (B.22) est connectée de manière fluidique et stérile à l'au moins une entrée du quatrième moyen de transfert de fluide (AS3), les au moins première et seconde sorties du quatrième moyen de transfert de fluide (AS3) sont connectées de manière fluidique et stérile à l'ouverture d'entrée de chacun des septième et huitième sacs d'échantillonnage (B.32, B.33), et la sortie du quatrième sac tampon (B.23) est connectée de manière fluidique et stérile à l'au moins une entrée du quatrième moyen pour Transfert de fluide (AS3); et

le procédé comprenant en outre les étapes suivantes:

- appliquer un champ acoustique à l'intérieur du canal du quatrième moyen de transmission de fluide (AS3) à l'aide du générateur d'ondes sonores ;
- transférer le contenu du quatrième sac d'échantillonnage (B.22) et du quatrième sac tampon (B.23) dans le quatrième moyen de transfert de fluide (AS3); et
- Recueillir le concentré de plaquettes dans le septième sac d'échantillonnage (B.32).

5. Procédé de préparation de produits sanguins à haut débit selon l'une quelconque des revendications **1** à **4,** dans lequel les moyens de transmission de fluide comprennent en outre au moins un générateur d'ondes sonores pour générer des ondes sonores avec une longueur d'onde λ dans le canal ; et dans lequel l'épaisseur du canal des moyens de transfert de fluide est égale à $\frac{\lambda}{2}$ ou à un multiple de $\frac{\lambda}{2}$.

6. Procédé de préparation de produits sanguins à haut débit selon l'une quelconque des revendications **1** à **5,** dans lequel le champ acoustique dans le canal des moyens de transmission de fluide (ASO, AS1, AS2, AS3) contient des ondes stationnaires acoustiques volumétriques.

7. Procédé de préparation de produits sanguins à haut débit selon l'une quelconque des revendications **1** à **6,** dans lequel le débit se trouve dans la plage allant de 0,5 ml/min. jusqu'à 100ml / min, préférentiellement de 0,5 ml / min. jusqu'à 20 ml / min.

8. Procédé A pour la préparation d'un débit élevé de produits sanguins selon l'une quelconque des revendications **1** à **7,** dans lequel rapport largeur / épaisseur du canal est supérieur à 2 ou le ratio de la longueur / épaisseur du canal est supérieur à 10.

9. Procédé de préparation de produits sanguins à haut débit selon l'une quelconque des revendications **1** à **8,** dans lequel l'épaisseur du canal est supérieure à 300 microns, de préférence allant de 375 microns à 750 microns.

10. Procédé de préparation de produits sanguins à haut débit selon l'une quelconque des revendications **1** à **9,** dans lequel la largeur du canal est supérieure à 10 millimètres.

11. Procédé de préparation de produits sanguins à haut débit selon l'une quelconque des revendications **1** à **10,** dans lequel la longueur du canal le long de l'axe longitudinal est supérieure à 10 centimètres.

12. Procédé de préparation de produits sanguins à haut débit selon l'une quelconque des revendications **1** à **11,** dans lequel le sac de collecte de fluide (B.00) comprend une ouverture d'entrée qui est connectée de manière fluidique et stérile à une aiguille de ponction veineuse.

13. Procédé de préparation d'un débit élevé de produits sanguins selon l'une quelconque des revendications **1** bis **12,** dans lequel les étapes consistant à appliquer un champ acoustique à l'intérieur du canal comprend l'étape consistant à coupler le générateur d'ondes acoustiques à la première paroi du canal, et dans lequel la conductivité acoustique du premier mur varie de 0,5 à 1.

14. Procédé de préparation de produits sanguins à haut débit selon l'une quelconque des revendications **1** à **13,** dans lequel la deuxième paroi du canal est un réflecteur, et dans lequel le coefficient de réflexion sonore de la seconde paroi varie de 0,5 à 1.

FIG. 1A

FIG. 1B

FIG. 2

FIG. 3

FIG. 4

**FIG. 5**

**FIG. 6**

**FIG. 7**

**FIG. 8**

**FIG. 9**

**FIG. 10**

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008181828 A
- US 20140230912 A